# EUROPÄISCHE PATENTSCHRIFT

(11) **EP 2 686 309 B1**
(45) Veröffentlichungstag und Bekanntmachung des Hinweises auf die Patenterteilung: **11.05.2016**
(21) Anmeldenummer: 12707814.5
(22) Anmeldetag: 12.03.2012
(51) Int. Cl.: C07D 271/08, C07D 413/12, C07D 413/14, C07D 417/14, A01N 43/836

(54) **HERBIZID-SAFENER-ZUSAMMENSETZUNGEN**
HERBICIDE SAFENER COMPOSITIONS
COMPOSITIONS D'HERBICIDES ET DE PHYTOPROTECTEURS

(30) Priorität: 15.03.2011 EP 11158261
(43) Veröffentlichungstag der Anmeldung: 22.01.2014
(73) Patentinhaber: Bayer Intellectual Property GmbH, 40789 Monheim (DE)
(72) Erfinder: KÖHN, Arnim, 55270 Klein-Winternheim (DE); VAN ALMSICK, Andreas, 61184 Karben (DE); AHRENS, Hartmut, 63329 Egelsbach (DE); ZIEMER, Frank, 65830 Kriftel (DE); DÖRNER-RIEPING, Simon, 61267 Neu-Anspach (DE); ROSINGER, Christopher, Hugh, 65719 Hofheim (DE); HACKER, Erwin, 88515 Langenenslingen (DE)
(74) Vertreter: BIP Patents
(86) Internationale Anmeldenummer: PCT/EP2012/054286
(87) Internationale Veröffentlichungsnummer: WO 2012/123420

(56) Entgegenhaltungen:
- EP-A2- 0 173 657
- WO-A1-2011/035874
- DE-A1-102004 035 136

## Beschreibung

Die vorliegende Erfindung betrifft agrochemisch wirksame Herbizid-Safener-Zusammensetzungen, Verfahren zu deren Herstellung sowie deren Verwendung zur Bekämpfung von Schadpflanzen.

Aus verschiedenen Schriften ist bekannt, dass bestimmte N-(1,2,5-Oxadiazol-3-yl)benzamide herbizide Eigenschaften besitzen. So sind in EP 0 173 657 A1 und in WO 2011/035874 A1 solche N-(1,2,5-Oxadiazol-3-yl)benzamide beschrieben, die ein breites Spektrum von Unkräutern bekämpfen. Allerdings sind diese Wirkstoffe zum Teil nicht voll verträglich mit einigen wichtigen Kulturpflanzen, wie Getreidearten, Mais oder Reis. Sie können deshalb in manchen Kulturen nicht so eingesetzt werden, dass die erwünschte breite herbizide Wirksamkeit gegenüber Schadpflanzen gewährleistet ist.

Aufgabe der vorliegenden Erfindung ist es daher, herbizide Mittel zu bereitzustellen, in welchen die Selektivität der oben genannten Herbizide gegenüber wichtigen Kulturpflanzen erhöht ist. Diese Aufgabe wird durch die nachfolgend beschriebenen erfindungsgemäßen Zusammensetzungen enthaltend Herbizide und Safener gelöst.

Gegenstand der vorliegenden Erfindung sind Zusammensetzungen, enthaltend Herbizid-Safener-Zusammensetzungen, enthaltend
(A) eine oder mehrere Verbindungen der Formel A1-62 oder A1-85 oder deren Salze, 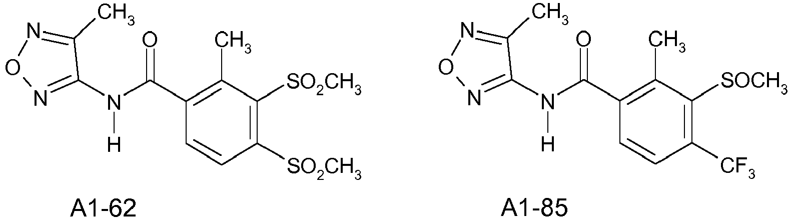 sowie
(B) einen oder mehrere Safener aus der Gruppe bestehend aus Mefenpyr-diethyl, Fenchlorazol(-ethylester), Isoxadifen-ethyl, Cloquintocet-mexyl, Benoxacor, Furilazol, Cyprosulfamide und Fluxofenim.

Die erfindungsgemäßen Herbizid-Safener-Kombinationen können zusätzliche weitere Komponenten, beispielsweise Pflanzenschutzmittelwirkstoffe anderer Art und/oder im Pflanzenschutz übliche Zusatzstoffe und/oder Formulierungshilfsmittel, enthalten oder zusammen mit diesen eingesetzt werden.

Die Herbizide A1-62 und A1-85 und die Safener (B) können auf bekannte Weise angewendet werden, beispielsweise gemeinsam (beispielsweise als Co-Formulierung oder als Tank-Mischung) oder auch zeitlich versetzt (Splitting), z.B. auf die Pflanzen, Pflanzenteile, Pflanzensamen oder die Fläche, auf der die Pflanzen wachsen. Möglich ist z.B. die Anwendung der Einzelwirkstoffe oder der Herbizid-Safener-Kombination in mehreren Portionen (Sequenzanwendung), z. B. nach Anwendungen im Vorauflauf, gefolgt von Nachauflauf-Applikationen oder nach frühen Nachauflaufanwendungen, gefolgt von Applikationen im mittleren oder späten Nachauflauf. Bevorzugt ist dabei die gemeinsame oder die zeitnahe Anwendung der Wirkstoffe der jeweiligen Kombination. Möglich ist auch die Anwendung der Einzelwirkstoffe oder der Herbizid-Safener-Kombination zur Saatgutbehandlung.

Gegenstand der vorliegenden Erfindung sind auch Herbizid-Safener-Zusammensetzungen, umfassend Stereoisomere und deren Gemische, die von den Formeln der Komponente B (Safener) umfasst sind. Solche Verbindungen der Formeln der Komponente B (Safener) enthalten beispielsweise ein oder mehrere asymmetrisch substituierte Kohlenstoff-Atome oder Sulfoxide. Die durch ihre spezifische Raumform definierten möglichen Stereoisomeren, wie Enantiomere und Diastereomere, sind alle von den Formeln der Komponente B (Safener) umfasst; insbesondere auch die racemischen Gemische und - soweit Enantiomere möglich sind - beide Enantiomere und insbesondere das jeweils biologisch wirksame Enantiomer. Die einzelnen Stereoisomeren können nach üblichen Methoden aus Gemischen der Stereoisomeren erhalten oder auch durch stereoselektive Reaktionen in Kombination mit dem Einsatz von stereochemisch reinen Ausgangs- oder Hilfsstoffen hergestellt werden.

Die Aufwandmenge der Herbizide der Formel A1-62 oder A1-85 kann mit den äußeren Bedingungen wie Temperatur, Feuchtigkeit, der Art des verwendeten Herbizids in einem weiten Bereich variieren, beispielsweise zwischen 0,001 g und 2000 g a.i./ha (ai/ha bedeutet dabei im folgenden .Aktivsubstanz pro Hektar" = bezogen auf 100%igen Wirkstoff).

Bei Anwendungen mit Aufwandmengen von 0,01 g bis 1000 g a.i./ha der Herbizide der Formel A1-62 oder A1-85 wird im Vor- und Nachauflaufverfahren ein relativ breites Spektrum an Schadpflanzen bekämpft, z.B. annuellen und perennierenden mono- oder dikotylen Unkräutern sowie an unerwünschten Kulturpflanzen. Bei den erfindungsgemäßen Kombinationen liegen die Aufwandmengen in der Regel niedriger, z. B. im Bereich von 0,1 g bis 800 g a.i./ha, vorzugsweise 1 g bis 500 g a.i./ha, besonders bevorzugt 10 g bis 400 g a.i./ha.

Die Herbizide der Formel A1-62 oder A1-85 sind zur Bekämpfung von Schadpflanzen, z.B. in Pflanzenkulturen geeignet, beispielsweise in wirtschaftlich bedeutenden Ackerbaukulturen z.B. monokotylen Ackerbaukulturen wie Getreide (z.B. Weizen, Gerste, Roggen, Hafer), Reis, Mais, Hirse, oder dikotylen Ackerbaukulturen wie Zuckerrübe, Raps, Baumwolle, Sonnenblume und Leguminosen z.B. der Gattungen Glycine (z.B. Glycine max. (Soja) wie nicht-transgene Glycine max. (z.B. konventionelle Sorten wie STS-Sorten) oder transgene Glycine max. (z.B. RR-Soja oder LL-Soja) und deren Kreuzungen), Phaseolus, Pisum, Vicia und Arachis, oder Gemüsekulturen aus verschiedenen botanischen Gruppen wie Kartoffel, Lauch, Kohl, Karotte, Tomate, Zwiebel, sowie Dauer- und Plantagenkulturen wie Kern- und Steinobst, Beerenobst, Wein, Hevea, Bananen, Zuckerrohr, Kaffee, Tee, Citrus, Nussplantagen, Rasen, Palmenkulturen und Forstkulturen. Für die Anwendung der erfindungsgemäßen Herbizid-Safener-Kombinationen (A)+(B) sind diese Kulturen ebenfalls bevorzugt, besonders bevorzugt ist der Einsatz in Getreide (z.B. Weizen, Gerste, Roggen, Hafer), Reis, Mais, Hirse, Zuckerrübe, Zuckerrohr, Sonnenblume, Raps und Baumwolle. Die Herbizid-Safener-Kombinationen (A)+(B) sind auch einsetzbar in toleranten und nicht toleranten Mutantenkulturen und toleranten und nicht toleranten transgenen Kulturen, vorzugsweise von Mais, Reis, Getreide, Raps und Soja, z.B. solche die gegen Imidazolinon-Herbizide, Atrazin, Glufosinate oder Glyphosate resistent sind.

Die Verbindungen der Formel A1-62 und A1-85 sind aus WO 2011/035874 A1 bekannt und können nach den dort beschriebenen Verfahren erhalten werden.

Unter den als Komponente (B) enthaltenen Safenern werden Verbindungen verstanden, die geeignet sind, phytotoxische Wirkungen von Pflanzenschutzmittelwirkstoffen wie Herbiziden an Kulturpflanzen zu reduzieren.

Im Rahmen der vorliegenden Erfindung werden die Verbindungen der Formel A1-62 und A1-85 mit folgenden Safener-Verbindungen kombiniert:
Mefenpyr-diethyl (S1-1), Fenchlorazol(-ethylester) (S1-7), Isoxadifen-ethyl (S1-11), Cloquintocet-mexyl (S2-1), Benoxacor (S3-4), Furilazol (S3-10), Cyprosulfamide (S4-1) und Fluxofenim (S11-2).

Die Safener (B) eignen sich zur Reduktion phytotoxischer Effekte, die beim Einsatz von Herbiziden der Formel A1-62 oder A1-85 in Nutzpflanzenkulturen auftreten können, ohne die Wirksamkeit dieser herbiziden Wirkstoffe gegen Schadpflanzen wesentlich zu beeinträchtigen. Hierdurch kann das Einsatzgebiet herkömmlicher Pflanzenschutzmittel ganz erheblich erweitert z.B. auf Kulturen, in denen bisher ein Einsatz der Herbizide nicht möglich oder nur beschränkt möglich war.

Die benötigten Aufwandmengen der Safener können je nach Indikation und verwendetem herbiziden Wirkstoff innerhalb weiter Grenzen schwanken und sind in der Regel im Bereich von 0,001 bis 5 kg, vorzugsweise 0,005 bis 2,5 kg und besonders 0,05 bis 0,5 kg Wirkstoff je Hektar.

Die herbiziden Wirkstoffe der Formel A1-62 oder A1-85 und die Safener (B) können zusammen (z.B. als fertige Formulierung oder im Tank-mix-Verfahren) oder in beliebiger Reihenfolge nacheinander ausgebracht werden, z.B. durch Sprüh-, Gieß- und Spritzanwendung oder durch Granulatstreuung. Das Gewichtsverhältnis Herbizid der Formel A1-62 oder A1-85 : Safener (B) kann innerhalb weiter Grenzen variieren und liegt vorzugsweise im Bereich von 1:10000 bis 10000:1, insbesondere von 1:1000 bis 1000:1 und ganz besonders von 1:20 bis 20:1. Die jeweils optimalen Mengen an Herbiziden der Formel A1-62 oder A1-85 und Safener (B) sind vom Typ des verwendeten Herbizids und des verwendeten Safeners sowie von der Art und dem Entwicklungsstadium des zu behandelnden Pflanzenbestandes abhängig und lassen sich von Fall zu Fall durch einfache, routinemäßige Vorversuche ermitteln.

Die in der erfindungsgemäßen Herbizid-Safener-Kombination enthaltenen Safener (B) können je nach ihren Eigenschaften zur Vorbehandlung des Saatgutes der Kulturpflanze (z.B. zur Beizung des Saatguts) verwendet werden oder vor der Saat in die Saatfurchen eingebracht oder zusammen mit dem Herbizid vor oder nach dem Auflaufen der Pflanzen angewendet werden. Vorauflaufbehandlung schließt sowohl die Behandlung der Anbaufläche (einschließlich eventuell auf der Anbaufläche befindlichen Wassers, z.B. bei Reisapplikationen) vor der Aussaat als auch die Behandlung der angesäten, aber noch nicht bewachsenen Anbauflächen ein.

Bevorzugt ist die gemeinsame Anwendung mit dem Herbizid. Hierzu können Tankmischungen oder Fertigformulierungen eingesetzt werden.

In einer bevorzugten Ausführungsform werden das Saatgut (z.B. Körner, Samen oder vegetative Vermehrungsorgane wie Knollen oder Sprossteile mit Knospen) oder Setzlinge mit den Safenern (B), gegebenenfalls in Kombination mit anderen agrochemischen Wirkstoffen, vorbehandelt. Zur Vorbehandlung des Saatguts können die Wirkstoffe z.B. durch Beizung an das Saatgut gebracht oder die Wirkstoffe und das Saatgut können in Wasser oder andere Lösungsmittel gegeben, und die Wirkstoffe z.B. durch Anlagerung oder Diffusion im Tauchverfahren oder durch Quellen oder Vorkeimen aufgenommen werden. Zur Vorbehandlung von Setzlingen können die jungen Pflanzen z.B. durch Spritzen, Tauchen oder Gießen mit den Safenern, gegebenenfalls in Kombination mit anderen agrochemischen Wirkstoffen, in Kontakt gebracht und anschließend verpflanzt und gegebenenfalls mit den Herbiziden A1-62 oder A1-85 nachbehandelt werden.

Die Saatgut- oder Setzlingsbehandlung kann mit den Safenern (B) alleine oder gemeinsam mit anderen agrochemischen Wirkstoffen - wie Fungiziden, Insektiziden oder Mitteln zur Pflanzenstärkung, Düngung oder zur Beschleunigung der Quell- und Keimungsvorgänge - erfolgen. Dabei können die Safener nach der Vorbehandlungsanwendung anschließend nochmals vor, nach oder gemeinsam mit einem oder mehreren Herbiziden der Formel A1-62 oder A1-85 eventuell auch in Kombination mit anderen bekannten Herbiziden angewandt werden. Durch die Vorbehandlung des Saatguts oder der Setzlinge kann eine verbesserte Langzeitwirkung der Safener erzielt werden.

Gegenstand der vorliegenden Erfindung ist somit weiterhin ein Verfahren zur Bekämpfung von unerwünschten Pflanzen in Pflanzenkulturen, das dadurch gekennzeichnet ist, dass die Komponenten A1-62 oder A1-85 und (B) der erfindungsgemäßen Herbizid-Safener-Kombination auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden, z.B. gemeinsam oder getrennt. Dabei können einer oder mehrere Safener (B) nach oder gleichzeitig mit dem oder den Herbizid(en) A1-62 oder A1-85 auf die Pflanzen, das Saatgut oder die Fläche, auf der die Pflanzen wachsen (z.B. die Anbaufläche), appliziert werden. In einer bevorzugten Ausführungsform werden die Safener (B) zur Saatgutbehandlung eingesetzt.

Unter unerwünschten Pflanzen sind alle Pflanzen zu verstehen, die an Orten wachsen, wo sie unerwünscht sind. Dies können z.B. Schadpflanzen (z.B. mono- oder dikotyle Unkräuter oder unerwünschte Kulturpflanzen) sein, z.B. auch solche, die gegen bestimmte herbizide Wirkstoffe wie Glyphosate, Atrazin, Glufosinate oder Imidazolinon-Herbizide resistent sind.

Monokotyle Unkräuter entstammen z.B. den Gattungen Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera. Dikotyle Unkräuter entstammen z.B. den Gattungen Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum, Euphorbia.

Bevorzugt wird in dem erfindungsgemäßen Verfahren eine wirksame Menge der Komponenten (A) und (B) zur Bekämpfung von Schadpflanzen angewendet in Pflanzenkulturen, beispielsweise in wirtschaftlich bedeutenden Ackerbaukulturen z.B. monokotylen Ackerbaukulturen wie Getreide (z.B. Weizen, Gerste, Roggen, Hafer), Reis, Mais, Hirse, oder dikotylen Ackerbaukulturen wie Zuckerrübe, Raps, Baumwolle, Sonnenblumen und Leguminosen z.B. der Gattungen Glycine (z.B. Glycine max. wie nicht-transgene Glycine max. (z.B. konventionelle Sorten wie STS-Sorten) oder transgene Glycine max. (z.B. RR-Soja oder LL-Soja) und deren Kreuzungen), Phaseolus, Pisum, Vicia und Arachis, oder Gemüsekulturen aus verschiedenen botanischen Gruppen wie Kartoffel, Lauch, Kohl, Karotte, Tomate, Zwiebel, sowie Dauer- und Plantagenkulturen wie Kern- und Steinobst, Beerenobst, Wein, Hevea, Bananen, Zuckerrohr, Kaffee, Tee, Citrus, Nussplantagen, Rasen, Palmenkulturen und Forstkulturen.

Gegenstand der Erfindung ist auch die Verwendung der erfindungsgemäßen Herbizid-Safener-Kombinationen zur Bekämpfung von unerwünschtem Pflanzenwuchs, vorzugsweise in Pflanzenkulturen.

Die erfindungsgemäßen Herbizid-Safener-Kombinationen können nach bekannten Verfahren z.B. als Mischformulierungen der Einzelkomponenten, gegebenenfalls mit weiteren Wirkstoffen, Zusatzstoffen und/oder üblichen Formulierungshilfsmitteln hergestellt werden, die dann in üblicher Weise mit Wasser verdünnt zur Anwendung gebracht werden, oder als sogenannte Tankmischungen durch gemeinsame Verdünnung der getrennt formulierten oder partiell getrennt formulierten Einzelkomponenten mit Wasser hergestellt werden. Ebenfalls möglich ist die zeitlich versetzte Anwendung (Splitapplikation) der getrennt formulierten oder partiell getrennt formulierten Einzelkomponenten. Möglich ist auch die Anwendung der Einzelkomponenten oder der Herbizid-Safener-Kombinationen in mehreren Portionen (Sequenzanwendung), z. B. nach Anwendungen im Vorauflauf, gefolgt von Nachauflauf-Applikationen oder nach frühen Nachauflaufanwendungen, gefolgt von Applikationen im mittleren oder späten Nachauflauf. Bevorzugt ist dabei die gemeinsame oder die zeitnahe Anwendung der Wirkstoffe der jeweiligen Kombination.

Die erfindungsgemäße Herbizid-Safener-Kombination kann auch zur Bekämpfung von Schadpflanzen in Kulturen von bekannten oder noch zu entwickelnden gentechnisch veränderten Pflanzen eingesetzt werden.

Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.

Bevorzugt ist die Anwendung der erfindungsgemäßen Herbizid-Safener-Kombinationen oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.
Vorzugsweise können die Verbindungen der Formel A1-62 und A1-85 als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualität auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Herbizid-Safener-Kombinationen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Herbizid-Safener-Kombinationen in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Herbizid-Safener-Kombinationen zur Bekämpfung von Schadpflanzen in transgenen Kulturpflanzen.

Bevorzugt ist die Anwendung der erfindungsgemäßen Kombinationen in wirtschaftlich bedeutenden transgenen Kulturen von Nutz- und Zierpflanzen, z. B. von Getreide (z.B. Weizen, Gerste, Roggen, Hafer), Hirse, Reis, Maniok und Mais oder auch Kulturen von Zuckerrübe, Baumwolle, Soja, Raps, Kartoffel, Tomate, Erbse und anderen Gemüsekulturen.

Gegenstand der Erfindung ist deshalb auch die Verwendung der erfindungsgemäßen Herbizid-Safener-Kombinationen zur Bekämpfung von Schadpflanzen in transgenen Kulturpflanzen oder Kulturpflanzen, die Toleranz durch Selektionszüchtung aufweisen.

Die Herbizide A1-62 oder A1-85 und die Safener (B) können gemeinsam oder getrennt in übliche Formulierungen z.B. zur Sprüh-, Gieß-, Spritz- und Saatgutbeizanwendung übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen. Die Formulierungen können die üblichen Hilfs- und Zusatzstoffe enthalten.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel könne z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 Gew.-%.

Die Herbizide A1-62 oder A1-85 und die Safener (B) können als solche oder in ihren Formulierungen auch in Mischung mit anderen agrochemischen Wirkstoffen, wie bekannten Herbiziden, zur Bekämpfung von unerwünschtem Pflanzenwuchs, z.B. zur Unkrautbekämpfung oder zur Bekämpfung von unerwünschten Kulturpflanzen Verwendung finden, wobei z.B. Fertigformulierungen oder Tankmischungen möglich sind.

Auch Mischungen mit anderen bekannten Wirkstoffen wie Fungiziden, Insektiziden, Akariziden, Nematiziden, Schutzstoffen gegen Vogelfraß, Pflanzennährstoffen und Bodenstrukturverbesserungsmitteln sind möglich, ebenso mit im Pflanzenschutz üblichen Zusatzstoffen und Formulierungshilfsmitteln.

Die Herbizide A1-62 oder A1-85 und die Safener (B) können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht üblicherweise, z.B. durch Gießen, Spritzen, Sprühen, Streuen.

Die Wirkstoffe können auf die Pflanzen, Pflanzenteile, das Saatgut oder die Anbaufläche (Ackerboden) ausgebracht werden, vorzugsweise auf das Saatgut oder die grünen Pflanzen und Pflanzenteile und gegebenenfalls zusätzlich auf den Ackerboden. Eine Möglichkeit der Anwendung ist die gemeinsame Ausbringung der Wirkstoffe in Form von Tankmischungen, wobei die optimal formulierten konzentrierten Formulierungen der Einzelwirkstoffe gemeinsam im Tank mit Wasser gemischt und die erhaltene Spritzbrühe ausgebracht wird.

Eine gemeinsame Formulierung der erfindungsgemäßen Kombination an Wirkstoffen (A) und (B) hat den Vorteil der leichteren Anwendbarkeit, weil die Mengen der Komponenten bereits im optimalen Verhältnis zueinander eingestellt werden können. Außerdem können die Hilfsmittel in der Formulierung aufeinander optimal abgestimmt werden.

Als Kombinationspartner für die erfindungsgemäße Herbizid-Safener-Kombination in Mischungsformulierungen oder im Tank-Mix sind beispielsweise bekannte, vorzugsweise herbizide Wirkstoffe, die auf einer Inhibition von beispielsweise Acetolactat-Synthase, Acetyl-Coenzym-A-Carboxylase, PS I, PS II, HPPD, Phytoene-Desaturase, Protoporphyrinogen-Oxidase, Glutamine-Synthetase, Cellulosebiosynthese, 5-Enolpyruvylshikimat-3-phosphat-Synthetase beruhen, einsetzbar. Solche Verbindungen und auch andere einsetzbare Verbindungen mit teilweise unbekanntem oder anderem Wirkungsmechanismus sind z.B. in Weed Research 26, 441-445 (1986), oder in dem Handbuch "The Pesticide Manual", 12. Auflage 2000, oder 13. Auflage 2003 oder 14. Auflage 2006/2007, oder in dem entsprechenden "e-Pesticide Manual", Version 5.0 (2008-10), jeweils herausgegeben vom British Crop Protection Council, (im Folgenden auch kurz "PM"), und dort zitierter Literatur beschrieben. Listen von "Common names" sind auch in "The Compendium of Pesticide Common Names" im Internet verfügbar. Als literaturbekannte Herbizide, die mit den erfindungsgemäßen Herbizid-Safener-Zusammensetzungen kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet und umfassen stets sämtliche Anwendungsformen wie Säuren, Salze, Ester und Isomere wie Stereoisomere und optische Isomere. Dabei sind eine und zum Teil auch mehrere Anwendungsformen genannt):
2,4-D, Acetochlor, Acifluorfen, Acifluorfen-sodium, Aclonifen, Alachlor, Alloxydim, Alloxydim-sodium, Ametryn, Amicarbazone, Amidosulfuron, Aminopyralid, Amitrole, Anilofos, Asulam, Atrazine, Azafenidin, Azimsulfuron, Beflubutamid, Benazolin, Benazolin-ethyl, Benfuresate, Bensulfuron-methyl, Bentazone, Benzfendizone, Benzobicyclon, Benzofenap, Bifenox, Bilanafos, Bispyribac-natrium, Bromacil, Bromobutide, Bromofenoxim, Bromoxynil, Butachlor, Butafenacil, Butenachlor, Butralin, Butroxydim, Butylate, Cafenstrole, Carbetamide, Carfentrazone-ethyl, Chlomethoxyfen, Chloridazon, Chlorimuron-ethyl, Chlornitrofen, Chlortoluron, Chlorsulfuron, Cinidon-ethyl, Cinmethylin, Cinosulfuron, Clefoxydim, Clethodim, Clodinafop-propargyl, Clomazone, Clomeprop, Clopyralid, Cloransulam-methyl, Cumyluron, Cyanazine, Cyclosulfamuron, Cycloxydim, Cyhalofop-butyl, Desmedipham, Dicamba, Dichlobenil, Dichlorprop, Dichlorprop-P, Diclofop-methyl, Diclosulam, Difenzoquat, Diflufenican, Diflufenzopyr, Dikegulac-sodium, Dimefuron, Dimepiperate, Dimethachlor, Dimethametryn, Dimethenamid, Triaziflam, Diquatdibromide, Dithiopyr, Diuron, Dymron, EPTC, Esprocarb, Ethalfluralin, Ethametsulfuron-methyl, Ethofumesate, Ethoxyfen, Ethoxysulfuron, Etobenzanid, Fenoxaprop-ethyl, Fenoxaprop-P-ethyl, Fentrazamide, Flamprop-M-isopropyl, Flamprop-M-methyl, Flazasulfuron, Florasulam, Fluazifop, Fluazifop-butyl, Fluazifop-P-butyl, Fluazolate, Flucarbazone-sodium, Flucetosulfuron, Fluchloralin, Flufenacet, Flufenpyr, Flumetsulam, Flumiclorac-pentyl, Flumioxazin, Fluometuron, Fluorchloridone, Fluorglycofen-ethyl, Flupoxam, Flupyrsulfuron-methyl-sodium, Fluridone, Fluroxypyr, Fluroxypyr-butoxypropyl, Fluroxypyr-meptyl, Flurprimidol, Flurtamone, Fluthiacet-methyl, Fomesafen, Foramsulfuron, Glufosinate, Glufosinate-P, Glufosinate-ammonium, Glufosinate-P-ammonium, Glufosinate-P-sodium, Glyphosate, Halosulfuron-methyl, Haloxyfop, Haloxyfop-ethoxyethyl, Haloxyfopmethyl, Haloxyfop-P-methyl, Hexazinone, Imazamethabenz-methyl, Imazamox, Imazapic, Imazapyr, Imazaquin, Imazethapyr, Imazosulfuron, Indanofan, Indaziflam, Iodosulfuron-methyl-natrium, loxynil, Isoproturon, Isouron, Isoxaben, Isoxachlortole, Isoxaflutole, Ketospiradox, Lactofen, Lenacil, Linuron, MCPA, Mecoprop, Mecoprop-P, Mefenacet, Mesosulfuron-methyl, Mesotrione, Metamifop, Metamitron, Metazachlor, Methabenzthiazuron, Methiozolin, Methyldymron, Metobromuron, Metolachlor, Metosulam, Metoxuron, Metribuzin, Metsulfuron-methyl, Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonic acid, Pendimethalin, Pendralin, Penoxsulam, Pentoxazone, Pethoxamid, Phenmedipham, Picloram, Picolinafen, Pinoxaden, Piperophos, Pretilachlor, Primisulfuron-methyl, Profluazol, Profoxydim, Prometryn, Propachlor, Propanil, Propaquizafop, Propisochlor, Propoxycarbazone-sodium, Propyrisulfuron, Propyzamide, Prosulfocarb, Prosulfuron, Pyraclonil, Pyraflufenethyl, Pyrazolate, Pyrazosulfuron-ethyl, Pyrazoxyfen, Pyribenzoxim, Pyributicarb, Pyridafol, Pyridate, Pyriftalid, Pyriminobac-methyl, Pyrithiobac-sodium, Quinclorac, Quinmerac, Quinoclamine, Quizalofop-ethyl, Quizalofop-P-ethyl, Quizalofop-P-tefuryl, Rimsulfuron, Sethoxydim, Simazine, Simetryn, S-Metolachlor, Sulcotrione, Sulfentrazone, Sulfometuron-methyl, Sulfosate, Sulfosulfuron, Tebuthiuron, Tepraloxydim, Terbuthylazine, Terbutryn, Thenylchlor, Thiazopyr, Thifensulfuronmethyl, Thiobencarb, Tiocarbazil, Tralkoxydim, Triallate, Triasulfuron, Tribenuronmethyl, Triclopyr, Tridiphane, Trifloxysulfuron, Trifluralin, Triflusulfuron-Methyl-und Tritosulfuron.

### Weitere mögliche Mischungspartner sind

Pyroxasulfone, Pyroxsulam, Orthosulfamuron, Pyrimisulfan, Prohexadione-Calcium, Bencarbazone, SYN-523, IDH-100, SYP-249, Monosulfuron, Ipfencarbazone (HOK-201), Pyribambenz-Isopropyl, Tefuryltrione, Bencarbazone, Tembotrione, Pyrasulfotole und Thiencarbazone-Methyl.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

### Biologische Beispiele:

Herbizidwirkung und Safenerwirkung im Nachauflauf Samen bzw. Rhizomstücke von monound dikotylen Schadpflanzen und von Kulturpflanzen werden in Torftöpfen in sandiger Lehmerde ausgelegt, mit Erde abgedeckt und im Gewächshaus unter guten Wachstumsbedingungen angezogen. Alternativ hierzu werden im Paddy-Reisanbau vorkommende Schadpflanzen in Töpfen kultiviert, in denen Wasser bis zu 2 cm über der Bodenoberfläche steht. Zehn bis zwanzig Tage nach der Aussaat werden die Versuchspflanzen im ein bis Dreiblattstadium behandelt. Die als wasserlösliche Pulver oder Suspensionen formulierten erfindungsgemässen Herbizid-Safener-Zusammensetzungen sowie in parallelen Versuchen die entsprechend formulierten Einzelwirkstoffe werden in verschiedenen Dosierungen mit einer Wasseraufwandmenge von umgerechnet 300 l/ha auf die grünen Pflanzenteile gesprüht und nach 2-3 Wochen Standzeit der Versuchspflanzen im Gewächshaus unter optimalen Wachstumsbedingungen die Wirkung der Präparate optisch im Vergleich zu unbehandelten Kontrollen bonitiert. Bei Reis oder bei Schadpflanzen, die im Reisanbau vorkommen, werden die Wirkstoffe auch direkt ins Bewässerungswasser gegeben (Applikation in Analogie zur sogenannten Granulatanwendung) oder auf Pflanzen und ins Bewässerungswasser gesprüht.
Die Versuche zeigen, dass durch den Einsatz der erfindungsgemäßen Herbizid-Safener-Zusammensetzungen die Schäden an Kulturpflanzen wie Mais, Reis, Weizen oder Gerste im Vergleich zur Anwendung der einzelnen Herbizide ohne Safener wesentlich - d.h. um 30% bis zu 100% - reduziert werden. Gleichzeitig wird die Wirkung des Herbizids an wirtschaftlich bedeutenden Schadpflanzen nicht oder nicht wesentlich beeinträchtigt, so dass eine gute herbizide Nachauflaufwirkung gegen ein breites Spektrum von Ungräsern und Unkräutern erreicht werden kann.

Beispielsweise konnte in Gerste für die Herbizid-Safener-Zusammensetzungen (A1-85)+(S1-1), (A1-85)+(S1-11), (A1-85)+(S4-1), (A1-85)+(S2-1), (A1-85)+(S3-4), (A1-85)+(S11-2), (A1-62)+(S1-1), (A1-62)+(S1-11), (A1-62)+(S4-1), (A1-62)+(S2-1), (A1-62)+(S3-4), (A1-62)+(S3-10) und A1-62)+(S11-2) eine gute Verträglichkeit gegenüber den Kulturpflanzen bei gleichzeitig guter herbizider Nachauflaufwirkung gegen ein breites Spektrum von Schadpflanzen erzielt werden.
In Weizen konnte beispielsweise für die Herbizid-Safener-Zusammensetzungen (A1-85)+(S1-1), (A1-85)+(S1-11), (A1-85)+(S4-1), (A1-85)+(S2-1), (A1-85)+(S3-4), (A1-85)+(S3-10), (A1-85)+(S11-2), (A1-62)+(S1-11), (A1-62)+(S4-1), (A1-62)+(S2-1), (A1-62)+(S3-4), (A1-85)+(S3-10) und (A1-62)+(S11-2) eine gute Verträglichkeit gegenüber den Kulturpflanzen bei gleichzeitig guter herbizider Nachauflaufwirkung gegen ein breites Spektrum von Schadpflanzen erzielt werden. In Mais konnte beispielsweise für die Herbizid-Safener-Zusammensetzungen (A1-85)+(S1-1), (A1-85)+(S1-11), (A1-85)+(S2-1) und (A1-85)+(S3-4) eine gute Verträglichkeit gegenüber den Kulturpflanzen bei gleichzeitig guter herbizider Nachauflaufwirkung gegen ein breites Spektrum von Schadpflanzen erzielt werden

Herbizidwirkung und Safenerwirkung im Vorauflauf
Samen bzw. Rhizomstücke von monound dikotylen Unkrautpflanzen und Kulturpflanzen wurden in Torftöpfen in sandiger Lehmerde ausgelegt und mit Erde abgedeckt. Die als wasserlösliche Pulver oder Suspensionen formulierten erfindungsgemässen Herbizid-Safener-Zusammensetzungen sowie in parallelen Versuchen die entsprechend formulierten Einzelwirkstoffe wurden dann in verschiedenen Dosierungen mit einer Wasseraufwandmenge von umgerechnet 600 bis 800 l/ha auf die Oberfläche der Abdeckerde appliziert. Nach der Behandlung wurden die Töpfe im Gewächshaus aufgestellt und unter guten Wachstumsbedingungen für die Unkräuter und die Kulturpflanzen gehalten. Die optische Bonitur der Pflanzenbzw. Auflaufschäden erfolgte nach dem Auflaufen der Versuchspflanzen nach einer Versuchszeit von 2 bis 4 Wochen im Vergleich zu unbehandelten Kontrollen.

In Mais konnte beispielsweise für die Herbizid-Safener-Zusammensetzungen (A1-85)+(S1-11), (A1-85)+(S4-1), (A1-85)+(S2-1), (A1-85)+(S3-4), (A1-85)+(S3-10), (A1-85)+(S11-2), (A1-85)+(S1-7), (A1-62)+(S1-1), (A1-62)+(S4-1), (A1-62)+(S2-1), (A1-62)+(S3-4), (A1-85)+(S3-10) (A1-62)+(S11-2) und A1-62)+(S1-7) eine gute Verträglichkeit gegenüber den Kulturpflanzen bei gleichzeitig guter herbizider Vorauflaufwirkung gegen ein breites Spektrum von Schadpflanzen erzielt werden.

### Saatgutbehandlung

Saatkörner von Kulturpflanzen wurden mit den als Suspensions- oder Emulsionskonzentraten formulierten Safenern und Wasser in Flaschen gemischt und gut geschüttelt, so dass die Saatkörner gleichmässig mit der Formulierung des jeweiligen Safeners beschichtet wurden. Die Saatkörner bzw. die aufgelaufenen Pflanzen wurden dann im Vorauflauf oder Nachauflaufverfahren mit Herbiziden behandelt. Dabei zeigten zahlreiche erfindungsgemäße Herbizid-Safener-Zusammensetzungen eine gute Verträglichkeit gegenüber den Kulturpflanzen bei gleichzeitig guter herbizider Wirkung gegen ein breites Spektrum von Schadpflanzen.

## Patentansprüche

1. Herbizid-Safener-Zusammensetzungen , enthaltend
(A) eine oder mehrere Verbindungen der Formel A1-62 oder A1-85 oder deren Salze, 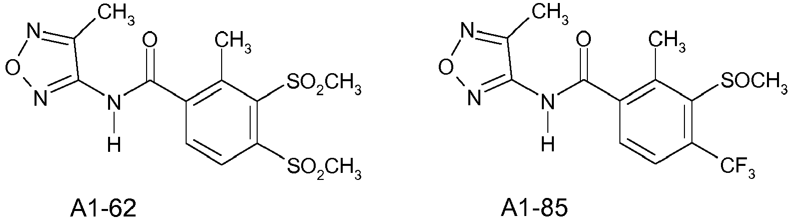 sowie
(B) einen oder mehrere Safener aus der Gruppe bestehend aus Mefenpyr-diethyl, Fenchlorazol(-ethylester), Isoxadifen-ethyl, Cloquintocet-mexyl, Benoxacor, Furilazol, Cyprosulfamide und Fluxofenim.

2. Verfahren zur Bekämpfung von Schadpflanzen in Kulturen, **dadurch gekennzeichnet, daß** man eine herbizid wirksame Menge einer Zusammensetzung gemäß Anspruch 1 auf die Schadpflanzen, Pflanzen, Pflanzensamen oder die Fläche, auf der die Pflanzen wachsen, aufbringt.

3. Verfahren gemäß Anspruch 2, **dadurch gekennzeichnet, daß** die Pflanzen aus der Gruppe Zuckerrohr, Mais, Weizen, Roggen, Gerste, Hafer, Reis, Sorghum, Baumwolle und Soja stammen.

4. Verfahren gemäß Anspruch 2 oder 3, **dadurch gekennzeichnet, daß** die Pflanzen gentechnisch verändert sind.

## Claims

1. Herbicide-safener compositions comprising
(A) one or more compounds of the formula A1-62 or A1-85 or salts thereof 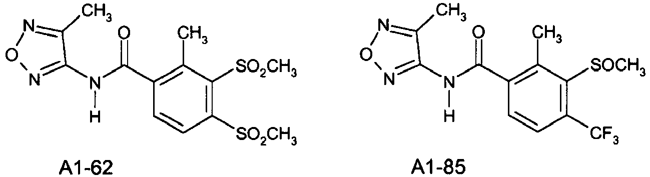 and also
(B) one or more safeners from the group consisting of mefenpyr-diethyl, fenchlorazole (ethyl ester), isoxadifen-ethyl, cloquintocet-mexyl, benoxacor, furilazole, cyprosulfamide and fluxofenim.

2. Method for controlling harmful plants in crops, **characterized in that** a herbicidally active amount of a composition according to Claim 1 is applied to the harmful plants, plants, plant seeds or the area on which the plants grow.

3. Method according to Claim 2, **characterized in that** the plants are from the group of sugarcane, corn, wheat, rye, barley, oats, rice, sorghum, cotton and soya.

4. Method according to Claim 2 or 3, **characterized in that** the plants have been genetically modified.

## Revendications

1. Compositions herbicide-agent protecteur, contenant :
(A) un ou plusieurs composés de formule A1-62 ou A1-85 ou leurs sels 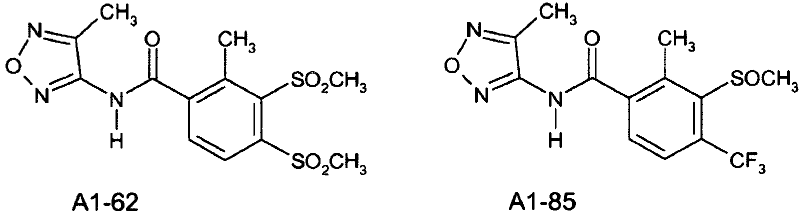 et
(B) un ou plusieurs agents protecteurs du groupe constitué par le méfenpyr-diéthyle, le fenchlorazole (ester éthylique), l'isoxadifène-éthyle, le cloquintocet-mexyle, le bénoxacor, le furilazole, le cyprosulfamide et le fluxofénim.

2. Procédé de lutte contre les plantes nuisibles dans des cultures, **caractérisé en ce qu'**une quantité herbicide efficace d'une composition selon la revendication 1 est appliquée sur les plantes nuisibles, les plantes, les graines des plantes ou la surface sur laquelle les plantes poussent.

3. Procédé selon la revendication 2, **caractérisé en ce que** les plantes sont issues du groupe constitué par la canne à sucre, le maïs, le blé, le seigle, l'orge, l'avoine, le riz, le sorgho, le coton et le soja.

4. Procédé selon la revendication 2 ou 3, **caractérisé en ce que** les plantes sont modifiées génétiquement.
